# EUROPEAN PATENT SPECIFICATION

(11) **EP 3 837 272 B1**
(45) Date of publication and mention of the grant of the patent: **26.02.2025**
(21) Application number: 18773251.6
(22) Date of filing: 16.08.2018
(51) Int. Cl.: C07K 17/14, C07K 1/113, C07K 14/195, C07K 1/13

(54) **METHOD FOR CONJUGATION OF BIOMOLECULES AND USE OF GOLD DONOR FOR BIOMOLECULAR COMPLEX FORMATION**
VERFAHREN ZUR KONJUGATION VON BIOMOLEKÜLEN UND VERWENDUNG EINES GOLDDONORS ZUR BILDUNG BIOMOLEKULARER KOMPLEXE
PROCÉDÉ DE CONJUGAISON DE BIOMOLÉCULES ET UTILISATION D'UN DONNEUR D'OR POUR LA FORMATION D'UN COMPLEXE BIOMOLÉCULAIRE

(43) Date of publication of application: 23.06.2021
(73) Proprietor: UNIWERSYTET JAGIELLONSKI, 31-007 Kraków (PL)
(72) Inventor: HEDDLE, Jonathan, Cockfield Bishop Auckland County Durham DL13 5HQ (GB); MALAY, Ali, Tokyo (JP)
(74) Representative: Witek, Rafal
(86) International application number: PCT/IB2018/056150
(87) International publication number: WO 2020/035716

(56) References cited:
- CN-A- 105 001 244
- US-A1- 2010 009 427
- ASAKO IGASHIRA-KAMIYAMA ET AL: "Rational creation of chiral multinuclear and metallosupramolecular compounds from thiol-containing amino acids", DALTON TRANSACTIONS, vol. 40, no. 28, 1 January 2011 (2011-01-01), pages 7249, XP055579179, ISSN: 1477-9226, DOI: 10.1039/c0dt01660h
- ALI D. MALAY ET AL: "Gold Nanoparticle-Induced Formation of Artificial Protein Capsids", NANO LETTERS, vol. 12, no. 4, 11 April 2012 (2012-04-11), US, pages 2056 - 2059, XP055579142, ISSN: 1530-6984, DOI: 10.1021/nl3002155
- JUDY CADDY ET AL: "Introduction of Phosphine-Gold(I) Precursors into a Cys-modified Enkephalin Neuropeptide as Part of Solid Phase Peptide Synthesis", ZEITSCHRIFT FUR NATURFORSCHUNG - SECTION B JOURNAL OF CHEMICAL SCIENCES, vol. 62, no. 3, 1 March 2007 (2007-03-01), DE, pages 460 - 466, XP055579156, ISSN: 0932-0776, DOI: 10.1515/znb-2007-0321
- BINDOLI A ET AL: "Thioredoxin reductase: A target for gold compounds acting as potential anticancer drugs", COORDINATION CHEMISTRY REVIEWS, ELSEVIER SCIENCE, AMSTERDAM, NL, vol. 253, no. 11-12, 1 June 2009 (2009-06-01), pages 1692 - 1707, XP026040264, ISSN: 0010-8545, [retrieved on 20090309], DOI: 10.1016/J.CCR.2009.02.026

## Description

### FIELD OF THE INVENTION

The present invention falls within the biochemistry field. It is related to the method for conjugation of free thiol group(s) containing biomolecules comprising a biomolecule reacting with a gold-donor agent in which a -S-Au-S- bond is formed, wherein the biomolecules are selected from the group consisting of polypeptides and proteins. Specifically, the method leads to a complex formation that is a protein cage.

### BACKGROUND

Protein complexes in nature represent important and highly sophisticated biological nanomachines and nano-structures. Large protein complexes in nature are typically constructed of a number of individual proteins held together by non-covalent interactions (i.e. hydrogen bonds, hydrophobic packing). This is particularly noticeable in protein cages such as capsids where multiple copies of identical protein subunits are held together in this way. In synthetic structural biology the ability to design and construct artificial protein assemblies may be useful, potentially allowing the introduction of properties an capabilities not present in nature. To this end new ways of connecting individual proteins together in defined ways is desirable.

Recently the inventors have studied such possibility using TRAP (trp RNA-binding attenuation protein) from *Geobacillus stearothermophilus* as a nanometric building block. This TRAP adopts an oligomeric ring structure of 11 subunits in the native state¹⁻⁵ and, along with a number of other ring proteins^{6,7}, has proven to be a useful bionano building block⁸⁻¹¹.

Having in mind disadvantages of known processes, the inventors have tried to find other methods for connecting protein subunits. Although there was some disclosure concerning binding two or other numbers of proteins via their cysteine SH groups, the inventors focused on this field taking into the consideration the use of gold as a "stitching" reagent.

The reactions of gold compounds with the -SH groups are well-known and described in the literature (for example, the MA Thesis of Stephanie A. Koening, The gold(I) mediated thoil/disulfide exchange reaction: a kinetic and mechanistic investigation, August 2007, Los Angeles, USA, Häkkinen H., The gold-sulfur interface at the nanoscale, Nat Chem. 2012 May 22;4(6):443-55 and Daniel MC, Astruc D., Gold nanoparticles: assembly, supramolecular chemistry, quantum-size-related properties, and applications toward biology, catalysis, and nanotechnology, Chem. Rev. 2004 Jan; 104(1):293-346 and the references therein).

The use of gold compounds to incorporate gold particles into nanostructures or providing nanoparticles as nanoclusters, protein cages for multiple applications, among others as a targeting molecule in delivery systems, is also well described in the literature as well as in patent documents and those ones are prior art for the present invention. For example, the International Application No PCT/KR2013/004454 describes a method for preparing a hyaluronic acid-gold nanoparticles/protein complex that can be used as a liver targeted drug delivery system, by surface modifying gold nanoparticles having excellent stability in the body with hyaluronic acid having biocompatibility, biodegradability and liver tissue-specific delivery properties, and binding protein drugs for treating liver diseases to the non-modified surface of the gold nanoparticles.

The US Patent Application No US 10/142,838 discloses the introduction of a precious metal atoms such as gold into a cage-like protein such as apoferritin by modifying the inner structure of a cage-like protein, and thus to form the precious metal - recombinant cage-like protein complex applicable to various microstructures.

The International Application No PCT/US2011/034190 discloses antibody-nanoparticle conjugates that include two or more nanoparticles (such as gold, palladium, platinum, silver, copper, nickel, cobalt, iridium, or an alloy of two or more thereof) directly linked to an antibody or fragment thereof through a metal-thiol bond.

Another example is US Patent Application No US 14/849,379 which discloses a recombinant self-assembled protein, comprising a target-oriented peptide fused to a self-assembled protein and a gold ion reducing peptide self-assembled.

The novel approach for use of gold compounds in building biological molecules for different purposes is shown also in the publication of A. D. Malay, et. al., Nanoletters, "Gold Nanoparticles-Induced Formation of Artificial Protein Capsid", where gold nanoparticles (GNP) are used as a catalyst for linking together ring-shaped TRAP monomers presumably by the S-Au-S bond formation though this was not determined in the above work. The use of GNP in the reaction is not desirable as 1.4 nm nanoparticles are known to be toxic ^{12,13} and may non-specifically bind to the resulting structures making purification of protein cage product from excess gold nanoparticles challenging and representing an obstacle to potential future *in vivo* applications. The present disclosure solves these problems.

There are many disclosures in the art concerning the mechanisms of Au-S bond formation, also for the biological structures formation. There are also data revealed in relation to the use of tri-R-phosphine gold chloride as an Au donor/catalyst for the reaction. It is also known in the art, that the cysteine SH groups, naturally occurred in the polypeptide chain, are used as a target for Au(I) atoms. Nevertheless, the SH blocking reactions using Au bearing compounds or the method of Au bearing markers incorporation on biological molecule surfaces for detection techniques, are mainly described in the art.

ASAKO IGASHIRA-KAMIYAMA ET AL, "Rational creation of chiral multinuclear and metallosupramolecular compounds from thiol-containing amino acids", DALTON TRANSACTIONS, (20110101), vol. 40, no. 28, discloses a method and use for conjugating free thiol groups of amino acids, leading to biomolecular complex formation, comprising a reaction to connect thiol-containing amino acids via reaction with gold in which a -S-Au-S- bond is formed.

CN105001244B discloses a triazole gold compound as well as a preparation method and application thereof.

ALI D. MALAY ET AL, "Gold Nanoparticle-Induced Formation of Artificial Protein Capsids", NANO LETTERS, US, (20120411), vol. 12, no. 4 discloses the gold nanoparticle-induced formation of artificial protein capsids using bacterial TRAP proteins.

JUDY CADDY ET AL, "Introduction of Phosphine-Gold(I) Precursors into a Cys-modified Enkephalin Neuropeptide as Part of Solid Phase Peptide Synthesis", ZEITSCHRIFT FUR NATURFORSCHUNG - SECTION B JOURNAL OF CHEMICAL SCIENCES, DE, (20070301), vol. 62, no. 3 discloses the use of halogen(triarylphosphine) gold (I) to form complexes with thiol-containing biomolecules such as Cys-modified neuropeptide.

In the present invention a new approach is realised - instead of gold nanoparticles - (triarylphosphine)gold(I) halide is used as a catalyst for bond formation between protein units self-assembling into the protein complex, wherein SH groups are within the moiety, preferably cysteine moiety, naturally occurred or artificially incorporated in the protein structure. This approach allows control of the assembly and disassembly of, in one embodiment, the capsid-like protein complex, that is innovative in the view of the state of the art.

### SUMMARY OF THE INVENTION

The subject matter of the invention is a method for conjugation of free thiol group(s) moiety(s) of biomolecules, leading to the biomolecular complex formation, comprising a reaction between biomoleculesand gold-donor agent in which -S-Au-S- bond is formed, wherein a the gold-donor agent is halogen(triarylphosphine)gold (I), wherein the biomolecules are selected from the group consisting of polypeptides and proteins.

Preferably complex is symmetric or asymmetric.

Preferably, in the method described above, the moiety is cysteine. Preferably, the cysteine moiety is a naturally occurring moiety in the biomolecule. Also preferably, the cysteine moiety is artificially introduced into the biomolecule.

Preferably in the gold-donor of the method that is halogen(triarylphosphine)gold (I):halogen is selected from the group comprising chloro, bromo, iodo, fluoro;aryl is selected from the group comprising unsubstituted phenyl- or ortho-, meta- or para- mono or polysubstituted phenyl.

More preferably gold-donor agent is chloro[diphenyl(3-sulfonatophenyl)phosphine]gold (I).

More preferably the gold-donor agent is chloro(triphenylphosphine)gold (I).

Preferably the method described above comprises following steps:
a. biomolecules preparation,
b. conjugation of biomolecules by reaction of biomolecules with gold-donor,
c. purification of conjugation product.

Preferably the biomolecules preparation is performed by biomolecule expression in a suitable expression system and purification of the expression product.

Preferably at least one cysteine is introduced into the biomolecule at the step a of the method described above.

Preferably conjugation is performed in aqueous solution, at room temperature, for up to 3 days and the molar ratio of biomolecule : gold-donor is typically in the range 3:1 to 1:4.

Preferably the purification of the conjugation product is performed by at least one of the methods selected from the group of filtration, crystallization, centrifugation, column chromatography.

Preferably the biomolecules complex is a protein cage. More preferably the biomolecule is TRAP protein. The TRAP protein complex preferably consists of 24 biomolecule units.

The subject matter of the invention is also use of halogen(triarylphosphine)gold (I) molecules as the gold-donor agent in the method for biomolecules complex formation according to the above described method.

Preferably the use consists of conjugation of free thiol group(s)moiety of biomolecules by a reaction in which -S-Au-S- bond is formed. Preferably complex is protein cage. More preferably protein is TRAP.

For the purpose of this description, the reaction connecting biomolecules via -S-Au-S- bonds is a reaction in which gold connects two -SH groups derived from two cysteines which are the amino acids of two biomolecules being connected into the complex. Preferably at least one - S-Au-S- linkage is formed between two biomolecules. In another embodiment, two or more - S-Au-S- linkages are formed. The amount of linkages depends on the amount of cysteines in the biomolecule and its availability - exposition for the gold-donor.

If no cysteine is present in the biomolecule, or they are present but not available for the reaction, -SH group, preferably as a group of cysteine, may be introduced into the biomolecule.

Introduction of cysteine can be carried out by any method known in the art. For example, but not limited to, the introduction of the cysteine is performed by methods known in the art, such as commercial gene synthesis or PCR-based site-directed mutagenesis using modified DNA primers. Above-mentioned methods are known by the persons skilled in the art and ready-to use kits with protocols are available commercially.

-SH moiety may be introduced into the biomolecule also by modification of other amino acids in the biomolecule i.e. by site-directed mutagenesis or by solid phase peptide synthesis.

"Unit", "subunit", "molecule", "biomolecule", "monomer" are used alternatively in the description and means one molecule which connects to another molecule for the complex formation.

"Complex", "assembly", "aggregate", are used alternatively in the description and means a superstructure constructed in by the reaction between biomolecules. It is formed by units connected with -S-Au-S- linkages. The amount of the units involved in the complex depends of the nature of the biomolecule. More specifically, it depends on the amount of the biomolecule and the amount of -SH groups present in the biomolecule.

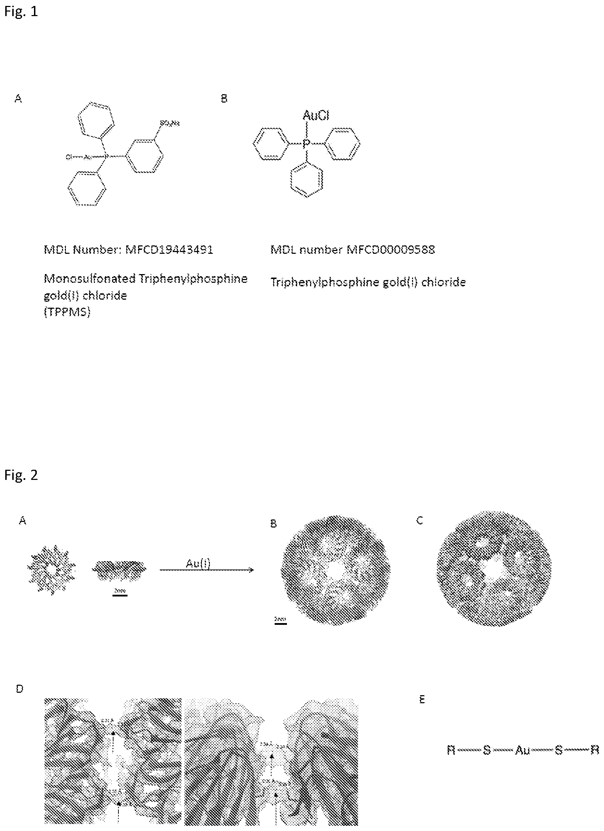

In order to carry out the connection reaction, i.e. using a source of Au(I) to link together two cysteines via S-Au-S bond formation, we first had to make and purify a monomer, and introduced, if relevant, reactable cysteine (Figure 2A), then carry out the reaction with gold-donor, for example - chloro[diphenyl(3-sulfonatophenyl)phosphine]gold (I), sodium salt hydrate (Au-TPPMS, MDL number MFCD19443491). As a result of the formation of -S-Au-S-bonds, a complex is assembled. The structure including S-Au-S bonds formation is confirmed using Cryo-EM, the presence of the S-Au-S bond is further confirmed by mass spectrometry measurements and the stability is confirmed by heat stability tests and others.

The stability of the complex obtained by the method according to the invention, in which the dative covalent bonds S-Au-S are formed, is, in general, more stable than a relevant complex, in which monomers are non-covalently linked.

The -S-Au-S bond is thought to have a mainly dative covalent character, compared to non-covalent hydrogen bonds, van der Waals type bonds that exists in protein-protein complexes known in the art. That is likely a factor as to why the stability of the complex obtained by the method according to the invention is high.

TRAP protein is a suitable biomolecule model for the method of the invention. This is likely due to its high intrinsic stability, toroid shape, lack of native cysteine residues (for easier control of the conjugation process) and availability of a residue that can be changed to cysteines with the resulting cysteine being in a suitable chemical and spatial environment suitable for S-Au-S bond formation.

### BRIEF DESCRIPTION OF THE FIGURES

**Fig. 1****:** illustrates examples of gold(I)-containing compounds for gold-stitching reaction.
**Fig 1A****.** Monosulfonated triphenylyphosphine gold(I) chloride.
**Fig. 1B****.** Triphenylyphosphine gold(I) chloride.
**Fig. 2****.** illustrates the gold-stitching reaction and an example of the resulting complex formed.
**Fig. 2A****.** Structure of a single TRAP ring (pdb 4v4f) shown in two mutually orthogonal views.
**Fig. 2B****.** Pseudo-atomic model of the structure of the hollow cage structure.
**Fig. 2C****.** The obtained cryo EM densities for the TRAP cage.
**Fig. 2D****.** Close up views of two neighbouring TRAP rings in the produced protein cage showing the presence of the bridging gold atom.
**Fig. 2E****.** Chemical bond formed between opposing cysteine side chains by the action of monosulfonated triphenylyphosphine gold(I) chloride, "R" refers to the remainder of the TRAP protein.
**Fig. 3A****:** illustrates LC-MS data for three forms of TRAP monomer: (from left) unliganded protein (dark grey), monomer bound to a single gold atom (grey), and monomer bound to a gold atom and TPPMS ligand (light grey).
**Fig. 3B****:** illustrates Native MS of intact TRAP cages performed at high collisional activation.
**Fig. 3C****:** Expansion of low-*m*/*z* region in **Fig 3B****.**
**Fig. 4** shows stability of protein complexes held together by the results of gold-stitching reaction.
**Fig. 4A** illustrates native PAGE gel showing the high thermal stability of the formed TRAP-cage.
**Fig. 4B** illustrates TEM images of the formed TRAP cage without heat treatment (scale bar 200 nm).
**Fig. 4C****:** shows TEM images of the formed TRAP cage after 3 hour incubation at 95 °C showing no significant degradation of the cage structure (scale bar 100 nm).

### EXAMPLES

### Techniques employed in the realisation of the invention

### Transmission electron microscopy (TEM)

Samples were typically diluted to a final protein concentration of 0.025 mg/ml, centrifuged briefly in a desktop centrifuge and the supernatant applied onto hydrophilized carbon-coated copper grids (STEM Co.), negatively stained with 4 % phospotungstic acid, pH 8, and visualized using a JEOLJEM-1230 80 kV instrument.

### Native PAGE

Samples were run on 3-12% native Bis-Tris gels following the manufacturer's recommendations (Life Technologies). Samples were mixed with 4x native PAGE sample buffer (200 mM BisTris, pH 7.2, 40% w/v Glycerol, 0.015% w/v Bromophenol Blue). As a qualitative guide to molecular weights of migrated bands, NativeMark unstained protein standard (Life Technologies) was used. Where blue native PAGE was performed, protein bands were visualized according to the manufacturer's protocol (Life Technologies), otherwise InstantBlue^{™} protein stain (Expedeon) was used.

### Electrothermal atomic absorption spectrometry (ETAAS)

A sample mass of approx. 2 mg was dissolved in 25 ml with 0.2 % HCl. The solution was then diluted 25 × before determination of total Au performed by an ETAAS spectrometer (PinAAcle 900Z, Perkin Elmer, Waltham, MA), with Zeeman background correction, at a wavelength of 242.80 nm (slit width of 0.7 nm). The measured volume of the sample solution was 10 µl and to each sample a mixture of matrix modifiers: 5 µg of Pd(NO₃)₂ and 3 µg of Mg(NO₃)₂ was added. 5 sets of measurements were carried out with each set consisting of 3 repeats.

### Protein expression and purification

In a typical purification, *E. coli* BL21(DE3) cells (Novagen) transformed with pET21b plasmid harboring the TRAP-CS gene were grown at 37 °C with shaking in 3 L of LB medium with 100 µg/ml ampicillin until OD₆₀₀ = 0.6, induced with 0.5 mM IPTG then further shaken for 4 h. Cells were harvested by centrifugation and the pellet kept at -80 °C until use. Cells were lysed by sonication at 4 °C in 50 ml of 50 mM Tris-HCl, pH 7.9, 50 mM NaCl in presence of proteinase inhibitors (Thermo Scientific) and presence or absence of 2 mM DTT, and lysates were centrifuged at 66,063 *g* for 0.5 h at 4 °C. The supernatant fraction was heated at 70 °C for 10 min, cooled to 4 °C, and centrifuged again at 66,063 g for 0.5 h at 4 °C. The supernatant fraction was purified by ion exchange chromatography on an ÄKTA purifier (GE Healthcare Life Sciences) using 4 × 5 ml HiTrap QFF columns with binding in 50 mM Tris-HCl, pH 7.9, 0.05 M NaCl, +/-2 mM DTT buffer and eluting with a 0.05 -1 M NaCl gradient. Fractions containing TRAP protein were pooled and concentrated using Amicon Ultra 10 kDa MWCO centrifugal filter units (Millipore) and the sample subjected to size exclusion chromatography on a HiLoad 16/60 Superdex 200 column in 50 mM Tris-HCl, pH 7.9, 0.15 M NaCl at room temperature. Protein concentrations were calculated using the BCA protein assay kit (Pierce Biotechnology).

### Liquid-chromatography mass spectrometry

TRAP cage sample was denatured in 50 mM Tris·HCl buffer (pH 8.0) with 8 M urea at 56 °C for 30 min, then buffer-exchanged to 50 mM Tris·HCl buffer (pH 8.0) using a centrifugal filtration device (Amicon 3 kDa MWCO, Millipore). For denaturing LC-MS analysis, the TRAP protein was desalted on a C18 pre-column (Acclaim PepMap100, C18, 300 µm × 1 cm; Thermo Scientific), then separated on a C18 column (Acclaim PepMap100, C18, 75 µm × 15 cm; Thermo Scientific) by Dionex UltiMate 3000 RSLCnano System connected to a hybrid LTQ Orbitrap XL mass spectrometer (Thermo Scientific) via a dynamic nanospray source. A binary buffer system was used, with buffer A 0.1% formic acid in H₂O, and buffer B 0.1% formic acid in acetonitrile. The proteins were separated at 25 °C with a gradient of 1 % to 90 % buffer B at a flow rate of 300 nL min⁻¹ over 60 min. The LTQ-Orbitrap XL was operated in positive ion mode with a nanoelectrospray voltage of 1.6 kV and capillary temperature of 275 °C. Survey full-scan MS spectra were acquired in the orbitrap (*m*/*z* 300-4000) with a resolution of 60000. The data were processed using Xcalibur 2.2 (Thermo Scientific).

### Native mass spectrometry

TRAP cage samples at 0.8 mg ml⁻¹ were prepared for native MS by buffer-exchanging into ammonium acetate (pH 6.9) using miniature spin columns (Micro Bio-Spin P-6, BioRad). This was performed in two steps: the first exchanged into 2.5 M ammonium acetate, the second into 200 mM ammonium acetate. Native MS experiments were performed using methods described previously¹⁴, employing a Q-ToF2 instruments (Waters Corp.), modified for the analysis of large protein ions¹⁵. Relevant instrument parameters were: nanoelectrospray capillary voltage: 1.9 kV; sample cone: 200 V; extractor cone: 10 V, acceleration into collision cell: 200 V. The collision cell was pressurized with argon at ≈35 µbar. Data was calibrated externally using MassLynx software (Waters Corp.), and are shown without background subtraction and minimal smoothing.

### Example 1

### TRAP complex preparation - reaction with Au-TPPMS

### (see Fig. 1)

### Gold compounds:

Chloro[diphenyl(3-sulfonatophenyl)phosphine]gold (I), sodium salt hydrate (Au-TPPMS, MDL number MFCD19443491) was purchased from STREM chemicals UK, limited and was made up to the desired concentration (typically 5mM) by dissolving in water. The gold nanoparticle (GNP) used was a diphenyl(m-sulfonatophenyl)phosphine-gold nanocluster with a 1-3 nm core diameter (MDL number MFCD17018839) from STREM Chemicals UK.

### TRAP Preparation:

The protein used that exemplifies the successful use of Au-TPPMS was TRAP protein with an introduced cysteine. Expression and purification of TRAP containing the mutation of residue lysine (K) number 35 to cysteine and an additional mutation of residue arginine (R) 64 to serine (S) (called "TRAP-CS") was similar to as described previously for TRAP-CS¹¹ (and as detailed above) with the notable change that TCEP (tris(2-carboxyethyl)phosphine) was not included in the lysis step. The final buffer was typically 20 mM Tris-HCl, pH 8.0, 0.15 M NaCl

*Reaction of modified TRAP protein with Au(I)-TPPMS.*

Purified TRAP protein was reacted with Au-TPPMS (Fig. 1A) in aqueous buffer at room temperature. S-Au-S bonds were formed in the reaction resulting in assembly of TRAP rings into the TRAP-cage which was then purified and further characterised.

Formation of TRAP-cage was carried out by mixing purified TRAP-CS and Au-TPPMS in aqueous solution. The exact concentrations of reactants were tailored for each reaction but were typically as follows: 1 mM TRAP-CS (8.3 mg ml⁻¹) and 1 mM Au-TPPMS in 50 mM Tris-HCl, pH 7.9, 0.15 M NaCl. Reactions were incubated for at least 3 days at room temperature. Formation of TRAP-cage was confirmed using TEM and native PAGE. Any precipitated material was removed by centrifugation at 12 045 × *g* for 5 min, and TRAP-cage was purified by size exclusion chromatography on either Superose 6 Increase 10/300 GL or HiPrep 16/60 Sephacryl S-500 HR column (GE Healthcare) or a HiLoad 16/600 Superdex 200pg. Fractions containing the cage protein were pooled, concentrated using Amicon Ultra 0.5 100 kDa MWCO, and protein concentrations were measured using the BCA protein assay (Pierce Biotechnology).

### Example 2

### TRAP complex preparation - reaction with Au(I)-triphenylphosphine

A similar reaction can be carried out with triphenylyphosphine gold(I) chloride (Au-TPP, Fig. 1B) instead of Au-TPPMS. Au-TPP is dissolved in DMSO and the reaction is carried out in 50 mM Tris, 150 mM NaCl, pH 7.9 and Au-TPP, with DMSO not exceeding 10% of the final volume and the TRAP monomer is present at a concentration of approximately 1mM. Ratios of Au-TPP to TRAP range from 4:2 to 4:3. This also results in TRAP-cage formation with the same structure as obtained in the reaction with Au-TPPMS. The amounts of the reagents were adjusted respectively. The ratio of the TRAP monomer/gold donor and conditions of the reaction were the same as in the reaction where Au-TPPMS was the gold-donor.

Two examples with different halogen(triarylphosphine)gold (I) gold-donor agents were performed above. It shows that halogen(triarylphosphine)gold (I) with different aryl moiety are suitable for the complex formation according to the invention.

### Example 3

### Confirmation of TRAP-complex structure using Cryo-EM

The initial (low resolution) cryo-EM structure of TRAP-cage was obtained using_cryo-EM single particle reconstruction techniques for TRAP-cage formed using GNPs. ^{10,11} and this structural data was used as an initial model for solving the high-resolution cryo-EM structure of TRAP-cage formed in the reaction with halogen(triarylphosphine)gold (I) obtained according to the invention.

Cryo-EM was used to solve the structure of the TRAP-cage to 3.9 Angstrom resolution. This was sufficient to show the arrangement of the 24 TRAP rings and to demonstrate the presence of a linking density (assigned to Au) between opposing cysteine side chains of the rings (See Fig. 2).

**Fig. 2****.** illustrates the gold-stitching reaction and an example of the resulting complex formed. **Fig 2A** shows structure of a single TRAP ring (pdb 4v4f) shown in two mutually orthogonal views. Mutated residues 35 and 64 are shown as spheres on each TRAP monomer with residue 35 being on the outer perimeter of the ring. Reaction with gold(I)-containing compound (arrow) results in structure shown in **Fig 2B** - pseudo-atomic model of the structure of the hollow cage structure. Here each of the 24 rings is shown in cartoon format with cysteines bridging the rings shown as sticks with spheres representing gold atoms between them. This model is built using structure illustrate in **Fig. 2C** that shows the obtained cryo EM densities. **Fig. 2D** is a close up views of two neighbouring TRAP rings in the produced protein cage showing the presence of the bridging gold atom. Cryo-EM map is shown as a grey net and protein is shown in cartoon format with cysteine residues shown as sticks. Four cysteine residues are highlighted by arrows and their bridging gold atoms are shown as spheres. Refined distances between Au-S linkages are indicated in **Fig. 2E** where chemical bond formed between opposing cysteine side chains by the action of monosulfonated triphenylyphosphine gold(I) chloride, "R" refers to the remainder of the TRAP protein, is shown.

### Cryo-EM single particle reconstruction of TRAP-cage formed using Au-TPPMS at higher resolution

Purified sample (3 µl of 0.89 mg ml⁻¹) formed using Au-TPPMS was applied to glow-discharged holey carbon grids (Quantifoil R 1.2/1.3, Mo 200 mesh) with a thin amorphous carbon film of ^{~}10 nm thickness over the holes and incubated for 30 s at 4 °C and 100% humidity. Grids were then blotted for 3.0 s and plunged into liquid ethane using a Vitrobot Mark IV (FEI). Data were recorded semi-automatically using the EPU software on a transmission electron cryo-microscope (FEI Titan Krios) operated at an accelerating voltage of 300 kV and at a nominal magnification of 75,000 ×. Images (0.91 Å/pixel) were recorded at applied underfocus values ranging from approximately -0.9 to -3.4 µm on a Falcon II direct electron detector (FEI) as 32 frames in 2.0 s exposure with a total electron dose of 40 electrons/Å². Data were subsequently aligned and summed using MotionCor2²¹ to obtain a final dose weighted image and then 2× binning was performed using the Bsoft program package,²² resulting in a pixel size of 1.82 Å for further image processing. Estimation of the contrast transfer function was performed using CTFFIND4.²³ Micrographs exhibiting poor power spectra based on the extent and regularity of the Thon rings were rejected (96 micrographs). Initially, approximately 2,000 particles were manually picked and subjected to reference-free two-dimensional (2D) classification using EMAN 2.1.¹⁸ Ten representative 2D class averages were selected as templates for automated particle picking using Gautomatch (http://www.mrc-Imb.cam.ac.uk/kzhang/). All subsequent processing steps were performed in RELION 2.0.²⁰ A total of 1,085,623 auto-picked particles from 10,290 micrographs were subjected to reference-free 2D classification to remove aberrant particles. Particles in 5 representative classes showing spherical shapes were selected (578,865 particles) for the following processes. The selected particles were subjected to three-dimensional (3D) classification into three classes using an angular sampling of 3.7° for 25 iterations without any symmetry (C1 symmetry), where the initial low-resolution structure as described above was used for the reference in the 3D classification after low-pass filtered to 60 Å. The particles (176,463 particles) in a class showing the most symmetrical cage structure with regular density distribution were selected for the following processes. However, although the density map clearly showed the overall TRAP-cage structure as a sphere with 24 11-membered rings, the structure at the level of the individual rings was curiously devoid of protein chiral features and showed mixed features of two mirrored protein structures, contrary to expectations from the protein structure previously determined by x-ray crystallography,²⁴ which is suggestive of the existence of chiral cage structures. Therefore, to separate the two chiral cage particles, we performed a second round of 3D classification into two classes using a finer angular sampling of 1.8° for 25 iterations without any symmetry (C1 symmetry). The resultant two maps clearly showed left-handed and right-handed structures at the level of the individual protein rings, respectively. Each structure (class I: 94,338 particles and class II: 82,125 particles) was refined individually with the C1 (asymmetric reconstruction), C4 and D4 symmetries. The resolutions of the class I were estimated to 3.9 (D4 sym.), 4.1 (C4 sym.), and 4.4 Å (C1 sym.) and the resolutions of the class II were estimated to 3.9 (D4 sym.), 4.2 (C4 sym.), and 4.5 Å (C1 sym.) by the gold-standard Fourier shell correlation (FSC = 0.143 criterion), after applying a soft spherical mask on the two reconstructions refined from the half of the data sets independently. According to the individual protein structures, the handedness of the class I map was corrected to the opposite one (resulting in class I: right-handed cage structures and class II: left-handed cage structures). The maps of the class I and II were sharpened with B-factors of -229 and -231 Å², respectively. Local resolution was estimated using ResMap.²⁵ Figures were prepared using UCSF Chimera.²⁶

### Structural refinement

The initial atomic coordinate model was based on the TRAP crystal structure (PDB accession 4V4F⁹), with the Cys³⁵ and Ser⁶⁴ substitutions modelled in Coot²⁷ to generate TRAP-CS ring structures. Note that residue positions have been renumbered from the initial deposited PDB to reflect the actual positions in the coding sequence of TRAP from *G. stearothermophilus* (e.g. the mutated Lys->Cys residue was assigned to residue number 37 in the original PDB file 4V4F but corresponds to residue number 35 in our analyses). Initial inspection of the density maps revealed areas of weak or missing density, and thus the structure of each TRAP subunit was truncated to residues 6-72; in addition residues 22-32 (corresponding to a loop that exhibits high flexibility in the *apo*-form of TRAP⁵) were omitted from the model to reflect this. Refinement of the LH and RH structures followed a similar regime. Twenty-four copies of TRAP-CS rings were initially fit into the cage density by rigid body refinement using Phenix real-space refinement.²⁸ Optimization of the original cryo-EM map voxel size using the high-resolution TRAP crystal structure²⁴ as a reference was performed as follows, in a manner analogous to previous reports.^{29,30} Comparison of cross-correlation scores of the fits between a simulated map of the TRAP-CS ring atomic model and the cryo-EM map at varying voxel scales (starting from the original 1.82 Å voxel⁻¹ and varying by 0.01 increments) was performed using Chimera, with the optimal results corresponding to a map scale of 1.74 Å voxel⁻¹. Similar results were obtained by performing rigid body refinement of individual subunits of 24 TRAP-CS rings onto the cryo-EM density at varying scales using Phenix.²⁸ Au^{I} atoms (120 in total) were docked manually into the prominent blobs of density between the Cys³⁵ side chains from neighbouring rings of the rigid-body fitted model, and subsequently 15 macro cycles of Phenix real-space refinement were run using the 1.74 Å voxel⁻¹ map, including rigid-body refinement, global minimization, a single round of simulated annealing, and adp refinement; restraints on the Au-S bond lengths and S-Au-S bond angles were applied during the later stages of refinement. Validation of the refined models was carried out using MolProbity.³¹ Analysis of interfacial contacts in the TRAP-cage models was performed using PDBePISA (http://www.ebi.ac.uk/pdbe/pisa/).³²

### Mass Spectometry:

Mass spectrometry was further used to support the presence of a gold atom linking TRAP monomers within the TRAP cage structure.

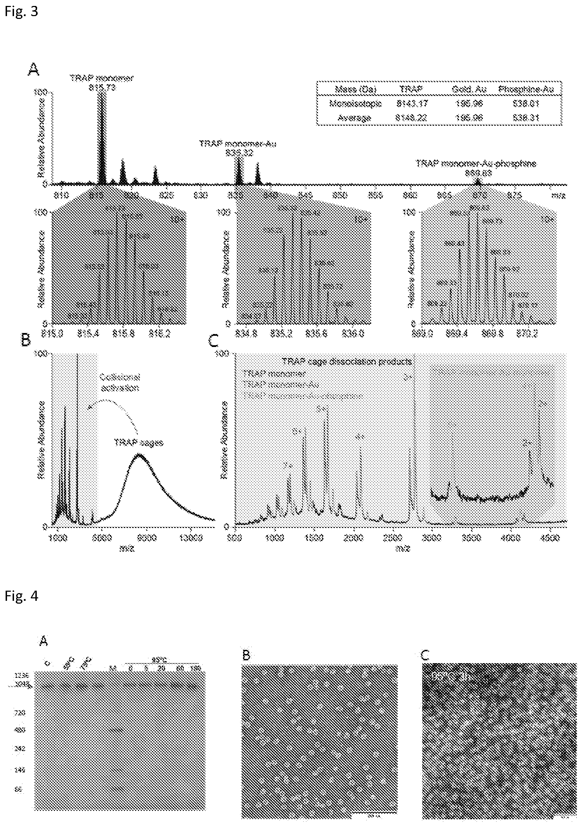

The results of mass spectrometry experiments are presented in the **Fig. 3A** that illustrates LC-MS data for three forms of TRAP monomer: (from left) unliganded protein (dark grey), monomer bound to a single gold atom (grey), and monomer bound to a gold atom and TPPMS ligand (light grey). Only 10+ charge states are show for clarity, and magnifications of the different peaks allows accurate mass determination for unambiguous assignment. The other, minor peaks correspond to salt adducts and/or other charge states. **Inset table** shows list of TRAP masses, and the mass additions expected due to the different modifications. These correspond very well to the masses measured, taking into account the 10 protons responsible for the 10+ charge state. **Fig. 3B** illustrates Native MS of intact TRAP cages performed at high collisional activation. Native MS of intact TRAP-cages performed at high collisional activation reveals a broad, unresolved region of signal at high *m*/*z,* and a series of peaks at low *m*/*z.* These features correspond to dissociation of intact cages, resulting in the release of cage fragments. illustrates expansion of low-*m*/*z* region. **Fig 3C** shows expansion of low-*m*/*z* region expressed in **Fig. 3B**, showing assignment of the various charge state series. Monomeric TRAP, in both modified and unmodified forms (greys, same colouring as **A**), are the major fragments observed. Mass spectra described above shows notably, peaks that can be assigned unambiguously to a TRAP dimer containing a single gold atom are observed, validating the TRAP-Au(I)-TRAP linkage hypothesis.

*Electrothermal atomic absorption spectrometry (ETAAS)* showed 112 ± 8 gold atoms per cage assembly, in close agreement with the predicted value of 120 (Table 1 below).

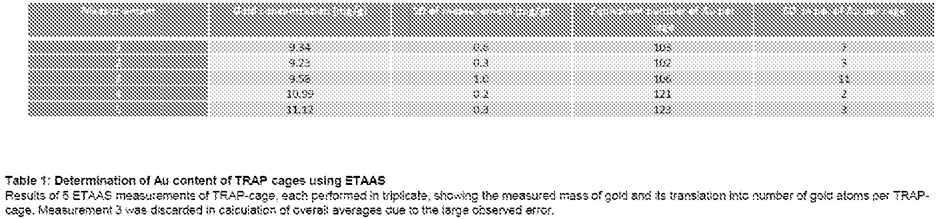

The Experiments above confirmed the structure of the TRAP-complex. The structure of the TRAP-complex obtained in the reaction with halogen(triarylphosphine)gold (I) is the same as obtained in the reaction with GNPs that was described before in the inventors' paper.

### Example 4

### Stability Tests of TRAP complex

Thermostability tests were performed as follows. Samples (7.5 µl) containing 1 µg TRAP cage protein in aqueous buffer were heated to 95 °C for different times (0-180 mins). After heating, samples were centrifuged at 10,000 rpm for 5 minutes in a bench-top centrifuge. Supernatants were taken and mixed with 2.5 µl of 4X NativePAGE sample buffer and the samples subjected to native PAGE analysis, the same sample were further analysed by TEM Typical results are shown in Fig. 4 A-C.

The stability of protein complexes held together by the results of gold-stitching reaction is presented in **Fig. 4****.**, in which . **Fig. 4A** illustrates Native PAGE gel showing the high thermal stability of the formed TRAP-cage. Samples were treated at the indicated temperatures for the indicated times before being applied to the gel. The band corresponding to the TRAP cage is indicated by the arrow. C = Control lane (no heat treatment). M=protein molecular weight marker (weights shown in kDa on left hand side of gel). **Fig. 4B** shows TEM images of the formed TRAP cage without heat treatment (scale bar 200 nm). **Fig. 4C** illustrates TEM images of the formed TRAP cage after 3 hour incubation at 95 °C showing no significant degradation of the cage structure (scale bar 100 nm).

### REFERENCES

1 Antson, A. A. et al. Structure of the trp RNA-binding attenuation protein, TRAP, bound to RNA. Nature 401, 235-242 (1999).
2 Antson, A. A. et al. The structure of trp RNA-binding attenuation protein. Nature 374, 693-700 (1995).
3 Chen, X. et al. Regulatory features of the trp operon and the crystal structure of the trp RNA-binding attenuation protein from Bacillus stearothermophilus. J. Mol. Biol. 289, 1003-1016, doi:10.1006/jmbi.1999.2834 S0022283699928346 [pii] (1999).
4 Watanabe, M. et al. The nature of the TRAP-Anti-TRAP complex. Proc. Natl. Acad. Sci. USA 106, 2176-2181 (2009).
5 Malay, A. D., Watanabe, M., Heddle, J. G. & Tame, J. R. H. Crystal structure of unliganded TRAP: implications for dynamic allostery. Biochem. J. 434, 429-434 (2011).
6 Ballister, E. R., Lai, A. H., Zuckermann, R. N., Cheng, Y. & Mougous, J. D. In vitro self-assembly of tailorable nanotubes from a simple protein building block. Proc. Natl Acad. Sci. USA 105, 3733-3738 (2008).
7 Wason, A., Pearce, F. G., Gerrard, J. A. & Mabbutt, B. C. Archaeal Lsm rings as stable self-assembling tectons for protein nanofabrication. Biochem. Biophys. Res. Commun. 489, 326-331, doi:10.1016/j.bbrc.2017.05.129 (2017).
8 Miranda, F. F. et al. A Self-Assembled Protein Nanotube with High Aspect Ratio. Small 5, 2077-2084 (2009).
9 Nagano, S. et al. Understanding the Assembly of an Artificial Protein Nanotube. Adv. Mater. Interfaces 3 (2016).
10 Malay, A. D. et al. Gold Nanoparticle-Induced Formation of Artificial Protein Capsids. Nano Lett. 12, 2056-2059 (2012).
11 Imamura, M. et al. Probing structural dynamics of an artificial protein cage using high-speed atomic force microscopy. Nano Lett. 15, 1331-1335, doi:10.1021/nl5045617 (2015).
12 Pan, Y. et al. Gold Nanoparticles of Diameter 1.4 nm Trigger Necrosis by Oxidative Stress and Mitochondrial Damage. Small 5, 2067-2076, doi:10.1002/smll.200900466 (2009).
13 Pan, Y. et al. Size-Dependent Cytotoxicity of Gold Nanoparticles. Small 3, 1941-1949, doi:10.1002/smll.200700378 (2007).
14 Kondrat, F. D., Struwe, W. B. & Benesch, J. L. Native mass spectrometry: towards high-throughput structural proteomics. Methods in molecular biology 1261, 349-371, doi:10.1007/978-1-4939-2230-7_18 (2015).
15 Sobott, F., Hernandez, H., McCammon, M. G., Tito, M. A. & Robinson, C. V. A tandem mass spectrometer for improved transmission and analysis of large macromolecular assemblies. Anal Chem 74, 1402-1407 (2002).
16 Li, X. et al. Electron counting and beam-induced motion correction enable near-atomic-resolution single-particle cryo-EM. Nat. Methods 10, 584-590, doi:10.1038/nmeth.2472 (2013).
17 Yasunaga, T. & Wakabayashi, T. Extensible and object-oriented system Eos supplies a new environment for image analysis of electron micrographs of macromolecules. J. Struct. Biol. 116, 155-160 (1996).
18 Tang, G. et al. EMAN2: an extensible image processing suite for electron microscopy. J. Struct. Biol. 157, 38-46, doi:10.1016/j.jsb.2006.05.009 (2007).
19 Ludtke, S. J., Baldwin, P. R. & Chiu, W. EMAN: semiautomated software for high-resolution single-particle reconstructions. J. Struct. Biol. 128, 82-97 (1999).
20 Scheres, S. H. RELION: implementation of a Bayesian approach to cryo-EM structure determination. J. Struct. Biol. 180, 519-530, doi:10.1016/j.jsb.2012.09.006 (2012).
21 Zheng, S. Q. et al. MotionCor2: anisotropic correction of beam-induced motion for improved cryo-electron microscopy. Nat. Methods 14, 331-332, doi:10.1038/nmeth.4193 (2017).
22 Heymann, J. B. Bsoft: image and molecular processing in electron microscopy. J. Struct. Biol. 133, 156-169, doi:10.1006/jsbi.2001.4339 (2001).
23 Rohou, A. & Grigorieff, N. CTFFIND4: Fast and accurate defocus estimation from electron micrographs. J. Struct. Biol. 192, 216-221, doi:10.1016/j.jsb.2015.08.008 (2015).
24 Hopcroft, N. H. et al. The interaction of RNA with TRAP: the role of triplet repeats and separating spacer nucleotides. J. Mol. Biol. 338, 43-53 (2004).
25 Kucukelbir, A., Sigworth, F. J. & Tagare, H. D. Quantifying the local resolution of cryo-EM density maps. Nat. Methods 11, 63-65, doi:10.1038/nmeth.2727 (2014).
26 Pettersen, E. F. et al. UCSF Chimera--a visualization system for exploratory research and analysis. J. Comput. Chem. 25, 1605-1612, doi:10.1002/jcc.20084 (2004).
27 Emsley, P., Lohkamp, B., Scott, W. G. & Cowtan, K. Features and development of Coot. Acta. Cryst. D 66, 486-501, doi:10.1107/S0907444910007493 (2010).
28 Adams, P. D. et al. PHENIX: a comprehensive Python-based system for macromolecular structure solution. Acta. Cryst. D 66, 213-221, doi:10.1107/S0907444909052925 (2010).
29 Wang, Z. et al. An atomic model of brome mosaic virus using direct electron detection and real-space optimization. Nat. Commun. 5, 4808, doi:10.1038/ncomms5808 (2014).
30 Natchiar, S. K., Myasnikov, A. G., Kratzat, H., Hazemann, I. & Klaholz, B. P. Visualization of chemical modifications in the human 80S ribosome structure. Nature 551, 472-477, doi:10.1038/nature24482 (2017).
31 Chen, V. B. et al. MolProbity: all-atom structure validation for macromolecular crystallography. Acta. Cryst. D 66, 12-21, doi:10.1107/S0907444909042073 (2010).
32 Krissinel, E. & Henrick, K. Inference of macromolecular assemblies from crystalline state. J. Mol. Biol. 372, 774-797, doi:10.1016/j.jmb.2007.05.022 (2007).

## Claims

1. Method for conjugation of free thiol group(s) of moiety(s) of biomolecules, leading to the biomolecular complex formation, comprising a reaction to connect biomolecules via reaction with a gold-donor agent in which a -S-Au-S- bond is formed, **characterised in that** the gold-donor agent is halogen(triarylphosphine)gold (I), wherein the biomolecules are selected from the group consisting of polypeptides and proteins.

2. Method according to claim 1, wherein complex is symmetric or asymmetric.

3. Method according to claim 1, wherein the moiety is cysteine.

4. Method according to claim 3, wherein cysteine moiety is a naturally occurring moiety in the biomolecule or it is artificially introduced into the biomolecule.

5. Method according to claim 1, wherein in halogen(triarylphosphine)gold (I):
halogen is selected from the group comprising chloro, bromo, iodo, fluoro; and
aryl is selected from the group comprising unsubstituted phenyl- or ortho-, meta- or para-mono or polysubstituted phenyl.

6. Method according to claim 1, wherein gold-donor agent is chloro[diphenyl(3-sulfonatophenyl)phosphine]gold (I) or chloro(triphenylphosphine)gold (I).

7. Method according to claim 1, comprising following steps:
biomolecules preparation,
conjugation of biomolecules by reaction of biomolecules with gold-donor,
purification of conjugation product,
wherein preferably biomolecules preparation is performed by biomolecule expression in a suitable expression system and purification of the expression product, and/or
at least one cysteine is introduced into the biomolecule at the step a,
optionally wherein the conjugation is performed in aqueous solution, at room temperature, for up to 3 days and the molar ratio of biomolecule: gold-donor is typically in the range 3:1 to 1:4; or
optionally wherein the purification of the conjugation product is performed by at least one of the method selected from the group of filtration, crystallization, centrifugation and column chromatography.

8. Method according to claim 1, wherein the biomolecules complex is a protein cage and the protein complex consists of 24 biomolecule units.

9. Use of halogen(triarylphosphine)gold (I) molecules as the gold-donor agent in the method for biomolecules complex formation according to any of the claims above.

10. Use according to claim 9 wherein complex formation that is conjugation of free thiol group(s) of moiety(s) of biomolecules by a "stitching" reaction in which a S-Au-S- bond is formed.

11. Method according to claim 1 or use according to claim 9 wherein the complex is a protein cage, preferably the protein is TRAP.

## Patentansprüche

1. Verfahren zur Konjugation von freien Thiolgruppen von Struktureinheiten von Biomolekülen, was zur Bildung eines biomolekularen Komplexes führt, umfassend eine Reaktion zum Verbinden von Biomolekülen über eine Reaktion mit einem Golddonormittel, wobei eine Bindung -S-Au-S- entsteht, **dadurch gekennzeichnet, dass** es sich bei dem Golddonormittel um Halogen(triarylphosphin)gold(I) handelt, wobei die Biomoleküle aus der Gruppe ausgewählt sind, die aus Polypeptiden und Proteinen besteht.

2. Verfahren gemäß Anspruch 1, wobei der Komplex symmetrisch oder asymmetrisch ist.

3. Verfahren gemäß Anspruch 1, wobei es sich bei der Struktureinheit um Cystein handelt.

4. Verfahren gemäß Anspruch 3, wobei die Cystein-Struktureinheit eine in dem Biomolekül natürlich vorkommende Struktureinheit ist oder künstlich in das Biomolekül eingeführt ist.

5. Verfahren gemäß Anspruch 1, wobei in dem Halogen(triarylphosphin)gold(I) das Halogen aus der Gruppe ausgewählt ist, die aus Chloro, Bromo, lodo, Fluoro besteht; und das Aryl aus der Gruppe ausgewählt ist, die aus unsubstituiertem Phenyl oder ortho-, meta- oder para-mono- oder -polysubstituiertem Phenyl besteht.

6. Verfahren gemäß Anspruch 1, wobei es sich bei dem Golddonormittel um Chloro[diphenyl(3-sulfonatophenyl)phosphin]gold(I) oder Chloro(triphenylphosphin)gold(I) handelt.

7. Verfahren gemäß Anspruch 1, umfassend die folgenden Schritte:
Herstellen von Biomolekülen,
Konjugieren der Biomoleküle durch Reaktion der Biomoleküle mit einem Golddonor,
Reinigen des Konjugationsprodukts,
wobei vorzugsweise das Herstellen der Biomoleküle durch Expression des Biomoleküls in einem geeigneten Expressionssystem und Reinigen des Expressionsprodukts erfolgt und/oder
in Schritt a wenigstens ein Cystein in das Biomolekül eingeführt wird,
wobei gegebenenfalls das Konjugieren in wässriger Lösung bei Raumtemperatur während bis zu drei Tagen durchgeführt wird und das Stoffmengenverhältnis von Biomolekül : Golddonor typischerweise im Bereich von 3:1 bis 1:4 liegt; oder
wobei gegebenenfalls das Reinigen des Konjugationsprodukts durch wenigstens eines der Verfahren erfolgt, die aus der Gruppe Filtration, Kristallisation, Zentrifugation und Säulenchromatographie ausgewählt sind.

8. Verfahren gemäß Anspruch 1, wobei der Biomolekülkomplex ein Proteinkäfig ist und der Proteinkomplex aus 24 Biomoleküleinheiten besteht.

9. Verwendung von Halogen(triarylphosphin)gold(I)-Molekülen als Golddonormittel in dem Verfahren der Bildung von Biomolekülkomplexen gemäß einem der vorstehenden Ansprüche.

10. Verwendung gemäß Anspruch 9, wobei es sich bei der Komplexbildung um eine Konjugation von einer oder mehreren freien Thiolgruppen von einer oder mehreren Struktureinheiten von Biomolekülen durch eine "stitching"-Reaktion, bei der eine S-AuS-Bindung entsteht, handelt.

11. Verfahren gemäß Anspruch 1 oder Verwendung gemäß Anspruch 9, wobei der Komplex ein Proteinkäfig ist, vorzugsweise dass es sich bei dem Protein um TRAP handelt.

## Revendications

1. Procédé destiné à la conjugaison d'un/de groupe(s) thiol libre(s) de fragment(s) de biomolécules, amenant à la formation du complexe biomoléculaire, comprenant une réaction pour relier des biomolécules à l'aide d'une réaction avec un agent donneur d'or dans lequel une liaison S-Au-S est formée, **caractérisé en ce que** l'agent donneur d'or est l'halogène(triarylphosphine)or (I), dans lequel les biomolécules sont sélectionnées à partir du groupe constitué de polypeptides et de protéines.

2. Procédé selon la revendication 1, dans lequel le complexe est symétrique ou asymétrique.

3. Procédé selon la revendication 1, dans lequel le fragment est la cystéine.

4. Procédé selon la revendication 3, dans lequel le fragment cystéine est un fragment naturel dans la biomolécule ou est introduit artificiellement dans la biomolécule.

5. Procédé selon la revendication 1, dans lequel dans l'halogène(triarylphosphine)or (I) :
l'halogène est sélectionné à partir du groupe comprenant chloro, bromo, iodo, fluoro ; et
l'aryle est sélectionné à partir du groupe comprenant un phényle non substitué ou un phényle ortho-, méta- ou para-monosubstitué ou polysubstitué.

6. Procédé selon la revendication 1, dans lequel l'agent donneur d'or est le chloro[diphényl(3-sulfonatophényl)phosphine)or I ou le chloro(triphénylphosphine)or (I).

7. Procédé selon la revendication 1, comprenant les étapes suivantes :
préparation de biomolécules,
conjugaison de biomolécules par réaction de biomolécules avec un donneur d'or,
purification de produit de conjugaison,
dans lequel de préférence la préparation de biomolécules est réalisée par l'expression de biomolécules dans un système d'expression approprié et la purification du produit d'expression, et/ou
au moins une cystéine est introduite dans la biomolécule à l'étape a,
facultativement dans lequel la conjugaison est réalisée en solution aqueuse, à température ambiante, pendant jusqu'à trois jours et le rapport molaire de biomolécule/donneur d'or est typiquement dans la plage de 3:1 à 1:4 ; ou
facultativement dans lequel la purification du produit de conjugaison est réalisée par au moins un des procédés sélectionnés à partir du groupe de la filtration, de la cristallisation, de la centrifugation et de la chromatographie sur colonne.

8. Procédé selon la revendication 1, dans lequel le complexe de biomolécules est une cage de protéines et le complexe de protéines est constitué de 24 unités de biomolécules.

9. Utilisation de molécules d'halogène(triarylphosphine)or (I) en tant que l'agent donneur d'or dans le procédé destiné à la formation de complexe de biomolécules selon l'une quelconque des revendications ci-dessus.

10. Utilisation selon la revendication 9, dans laquelle la formation de complexe qui est la conjugaison d'un(de) groupe(s) thiol libre(s) de fragment(s) de biomolécules par une réaction de « couture » dans laquelle une liaison S-Au-S est formée.

11. Procédé selon la revendication 1 ou utilisation selon la revendication 9, dans lesquels le complexe est une cage de protéines, de préférence la protéine est TRAP.
